# EUROPEAN PATENT SPECIFICATION

(11) **EP 4 176 407 B1**
(45) Date of publication and mention of the grant of the patent: **08.04.2026**
(21) Application number: 21749148.9
(22) Date of filing: 22.07.2021
(51) Int. Cl.: G06T 7/30

(54) **SYSTEM AND METHOD FOR TIME-SERIES IMAGING**
SYSTEM UND VERFAHREN ZUR ZEITREIHENBILDGEBUNG
SYSTÈME ET PROCÉDÉ D'IMAGERIE PAR SÉRIE CHRONOLOGIQUE

(30) Priority: 22.07.2020 GB 202011330
(43) Date of publication of application: 10.05.2023
(73) Proprietor: Boston Scientific Scimed, Inc., Maple Grove, MN 55311 (US)
(72) Inventor: VAN HERK, Marcel, Manchester Oxford Road Manchester M13 9PL (GB); MCWILLIAM, Alan, Manchester Oxford Road Manchester M13 9PL (GB); BANFILL, Kathryn, Manchester Oxford Road Manchester M13 9PL (GB); FAIVRE-FINN, Corinne, Manchester New Road Manchester M13 9PL (GB); GOODING, Mark, Oxford Oxfordshire OX2 0HP (GB); BOUKERROUI, Djamal, Oxford Oxfordshire OX2 0HP (GB)
(74) Representative: Peterreins Schley
(86) International application number: PCT/EP2021/070621
(87) International publication number: WO 2022/018237

(56) References cited:
- US-A1- 2017 249 740
- WOLTHAUS J ET AL: "Reconstruction of a time-averaged midposition CT scan for radiotherapy planning of lung cancer patients using deformable registration", MEDICAL PHYSICS, AIP, MELVILLE, NY, US, vol. 35, no. 9, 11 August 2008 (2008-08-11), pages 3998 - 4011, XP012116232, ISSN: 0094-2405, DOI: 10.1118/1.2966347
- BREHM MARCUS ET AL: "Cardiorespiratory motion-compensated micro-CT image reconstruction using an artifact model-based motion estimation", MEDICAL PHYSICS, AIP, MELVILLE, NY, US, vol. 42, no. 4, 1 April 2015 (2015-04-01), pages 1948 - 1958, XP012195666, ISSN: 0094-2405, [retrieved on 19010101], DOI: 10.1118/1.4916083

## Description

This invention relates to the fields of medical imaging and radiotherapy planning. In particular, to the creation of a 3D medical image, from a 4D medical image that takes account of motion of the patient whilst the medical images are acquired.

### Background

In radiotherapy treatment planning it is necessary to take a volumetric (3D) medical image of the patient, on which the treatment plan can be designed, and the radiation dose delivery estimated. Such a 3D medical image is called the simulation image. The 3D medical image is an image comprising a volume and is typically made from multiple 2D scan images. The terminology scan image will be used to refer to an image as acquired by the medical imaging device. A 2D scan image is an image comprising a slice in a 2D plane. The thickness of the slice may be defined, and thus the image constitutes a volume in a medical image as a cross section of the patient, each element in a 2D medical image is a pixel, whereas each element of the 3D medical image is known as a voxel. A medical image may also be derived from a combination of multiple scan images, in which instance the terminology derived image will be used. Part of the radiotherapy treatment planning process involves the annotation of anatomical and target structures on the simulation image such that the estimated radiation dose delivery to each of the structures can be calculated and reported back to the clinical staff doing the treatment planning. In radiotherapy planning this simulation image is normally a CT scan image, but MRI scan image may also be used.

Capturing a simulation 3D scan image assumes that the patient does not move as the 2D scan images are acquired. For some regions of the patient's anatomy, such as the head and neck, this is a reasonable assumption - since the patient is typically held in place by immobilization devices. However, this assumption is incorrect for the thoracic region where the patient breaths and their heart beats, leading to motion in the thoracic region. Acquisition of an 3D scan image from this region as the thoracic region is moving, can lead to motion artifacts and blur within the scan image, making the anatomy that is shown in the scan image unclear, since the duration of the acquisition required to capture a thoracic region lasts for multiple breathing cycles and heart beats. To overcome this, it is possible to perform a gated acquisition whereby 2D scan images are only acquired at a particular time in the anatomic motion cycle based on the measurement of physical signal(s) related to the anatomic motion. This may be respiratory gated (i.e. 2D scan images acquired only at maximum inhale/exhale), or cardiac gated (2D scan images acquired at end-diastole/systole). Such methods use a measurement device to determine the location in the breathing cycle or cardiac cycle and trigger the imaging device to only capture 2D scan images at the correct point in the breathing or cardiac cycle [Keall et al. 2006]. This approach can generate 2D scan images with less motion artifacts, but it will take longer to acquire all of the required 2D scan images to create a 3D scan image - since 2D scan images are only captured during part of the motion cycle. Respiratory motion reduction can also be achieved by asking the patient to hold their breath as the 3D scan image is acquired - known as breath-hold imaging [Keall et al. 2006]. However, this can be challenging for patients with lung cancer who would be receiving radiotherapy.

Gating and breath-hold techniques result in 3D scan images with reduced motion artifacts and can be used for treatment planning. However, delivery of radiotherapy is time consuming, and would be more time-consuming if using gating during treatment delivery, whereby the radiation beam is only used to irradiate the tumour at the specific point in the breathing cycle [Keall et al. 2006]. Thus, this is not common practice. Instead, it is more commonplace to try to take account of motion during the treatment delivery by defining margins in which the target/tumour is expected to move. To do so, it is necessary to measure the motion in the thoracic region. To this end, normal clinical practice is to acquire a time-series volumetric (3D+T, or 4D) medical image. A 4D medical image usually comprise of a series of 3D volumetric images, where each 3D volumetric image represents a different phase of a respiratory or cardiac cycle (a specific time point in a time series) as indicated by a measurement device or inferred from indirect measurements. Typically, this would be a respiratory phase (as this is the larger source of motion), where each 2D scan image captured is assigned to the 3D volumetric image representing the phase closest in position in the breathing cycle.

The process of capturing a 4D medical image whereby 2D scan images are acquired throughout an anatomical motion cycle and sorted into membership of the 3D volumetric images representing the phases of the physiological cycle according to a signal that is measuring the motion process is known as binning, and the images have been binned into the appropriate motion phase bin. Therefore, the 3D medical images comprising the 4D medical image are not considered as 3D scan images, since they do not represent a contiguous set of 2D scan images in order of acquisition time, even though they are contiguous spatially. Rather, the terminology 3D phase image is used to indicate the 3D medical image comprises a set of 2D scan images sorted according to the phase of a cycle. Nevertheless, a 3D phase image represents a spatially contiguous set of 2D scan images to make up the volumetric image.

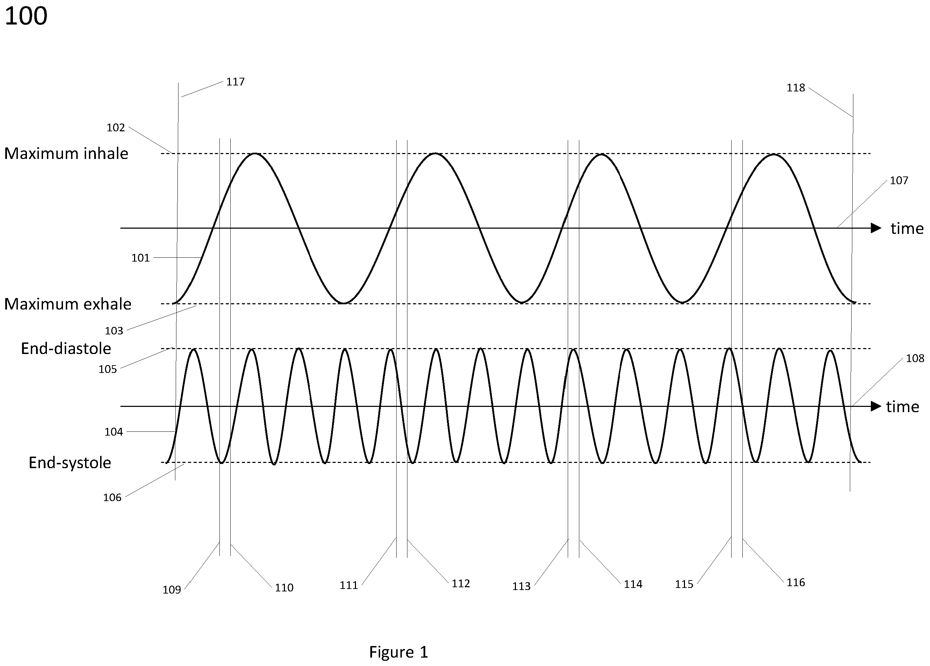

Figure 1 illustrates the changes in respiratory and cardiac cycles for a patient during the process 100 of acquiring a 4D medical image. A respiratory motion signal, shown by 101, is acquired using a standard measurement device. In this figure the respiratory motion signal is plotted against time, shown by axis 107, between maximum inhale, shown by dashed line 102, and maximum exhale, shown by dashed line 103. The acquisition of the imaging data occurs between the time point indicated by line 117 and the time point indicated by line 118, that is during four breath cycles. During this time a set of parallel 2D scan images are acquired for the patient moving along the patient axis. For example, for a scanning patient axis in the foot-to-head direction, at time point represented by line 117 the 2D scan image may be a cross-section through the patient just below the diaphragm in the abdomen, while at the time point indicated by line 118 the 2D scan image may be a cross-section through the shoulders. That is as the scanning time has progressed, the patient scan images acquired are from locations moving up the thorax of the patient. As time progresses scan images are acquired in spatial order between these locations. To produce the 4D medical image, these slices must be binned into 3D phases images. Each bin representing a different phase of the respiratory cycle. For example, the periods between the pairs of lines (109, 110), (111,112), (113,114), and (115,116) represent the same phase of the respiratory cycle, yet will contain different spatial locations within the patient, with the 2D scan image acquired between 109, 110, showing the region closest to the patient's diaphragm, and the 2D scan image acquired between 115 and 116 is an image acquired for a region closer to the patient's shoulders. Thus, these images are binned together to form a 3D phase image of the patient at that phase of the respiratory cycle. This binning is performed for a number, n, phases over the respiratory cycle, resulting in n 3D phase images covering the same spatial volume of the patient but acquired at different phases of the respiratory cycle. Thus, the use of the terminology "3D+T" to represent a 4D medical image comprising a set of 3D phases images may be regarded as misleading, since the time dimension, "+T", in this regard would refer to the phase of the breathing cycle rather than the temporal ordering of scan images according to acquisition time. Therefore, the use of the terminology "4D image" is preferred to represent a set of 2D scan images binned into a set of 3D phase images according to the phase of a motion cycle. Notwithstanding, the terminology "3D+T" may be used within the prior art to refer to a set of 3D phase images.

Starting from the 4D medical image, a static 3D derived image is created to serve as simulation CT for radiation treatment planning. Different prior art methods exist for the creation of the 3D derived image, and several approaches are used in clinical routine. These include, the Average Intensity Projection (AIP) image, The Maximum Intensity Projection (MIP) Image and the Mid-Position (Mid-P) Image [Keall et al. 2006, Wolthaus et al. 2008, Reconstruction of a time averaged midposition CT scan for radiotherapy planning of lung cancer patients using deformable registration, Wolthaus et al, Med. Phys. 35(9) p3998 Sept 2008].

The AIP technique consists of computing the 3D derived image defined as the voxel-wise average of the image intensity over all of the 3D phase images. The average is taken either as intensity mean value or as the intensity median value. In effect, the process of binning has been undone by the averaging, with each slice in the 3D derived image being a composite of all 3D phase images within the breathing cycle. Consequently, this technique leads to a very blurred 3D derived image due to ignoring the anatomical motion.

The MIP projection technique consists of computing the 3D derived image defined as the voxel-wise maximum of the image intensity over all the 3D phase images. Thus, similarly to the AIP, the process of binning has been undone. However, replacing the average operation with a max operation has the advantage of producing sharper images. Still, it has the drawback of producing an image that is not anatomically correct for any moving tissue due to ignoring the anatomical motion. For example, it will overestimate the size of a lung tumor, in CT imaging, if the tumor moves spatially with breathing motion, since in CT imaging the tumor is brighter than the lung parenchyma, therefore would represent the maximum intensity. Thus, by taking the voxel-wise maximum over all 3D phase images, the tumor is shown at all locations that it will appear during the breathing cycle.

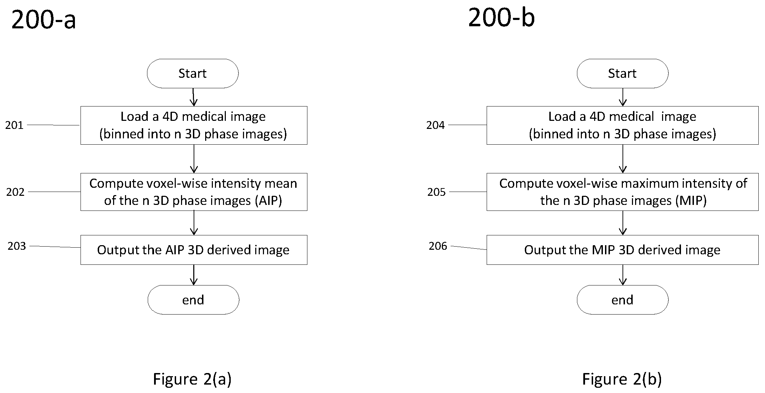

The AIP and the MIP are two techniques that creates a 3D derived image from a 4D medical image without any motion compensation. The flow diagrams of the two methods are shown in figure 2. The method detailed in figure 2 (a) starts by loading n 3D phase images of a 4D medical image 201, followed by the computation of a voxel-wise intensity mean across the n 3D phase images 202 and outputs an AIP 3D derived image 203. The method detailed in figure 2 (b) starts by loading n 3D phase images of a 4D medical image 204, followed by the computation voxel-wise maximum intensity across the n 3D phase images 205 and outputs a MIP 3D derived image 206.

Thus, both the pre-existing techniques of AIP and the MIP suffer from the effects tissue motion. Nevertheless, the extent of the motion of a tumour can be visualized, thus the target region for radiotherapy can be defined to encompass the motion of the tumour. However, this has the consequence that additional healthy tissue is being irradiated since the tumour does not occupy the full extent indicated on the AIP or MIP derived images at any point in time.

An alternative prior art technique to the AIP image, is to compute the average intensity image of the 3D phase images after a correction of the dominant tissue motion captured by the 4D imaging process. The dominant motion in the thorax is the respiratory motion. The correction of the dominant tissue motion is achieved using image registration. Various approaches to image registration are known [Maintz and Viergever 1998, Murphy, K et al. 2011, Brock et al. 2017]. Generally, a deformable image registration would be used, but local affine or rigid registration may also be considered. The motion correction aims at aligning all the 3D phase images to what is called the Mid-Position location, estimated as the geometric time-weighted mean anatomical position [Wolthaus et al. 2008]. The aim is to estimate a location from which the tumor motion is minimised in order to reduce the planning margins. Having already acquired a 4D medical image, it consists of the following steps [Wolthaus et al. 2008]:
1. Choose a reference 3D phase image (one of the 3D phase images from the 4D medical image).
2. Register each of the remaining 3D phase images of the 4D medical image to the reference 3D phase image
3. Compute the voxel-wise average Registration Vector Field (RVF) and its inverse. Starting from the reference 3D phase image location, the mid-position location is defined by the mean RVF. Note that the inverse of the mean RVF is needed to resample the reference 3D phase image to the mid-position location.
4. Resample each of the 3D phase images to the mid-position location.
5. Compute a voxel wise combination of the resampled 3D phase images to construct the mid-position derived image.

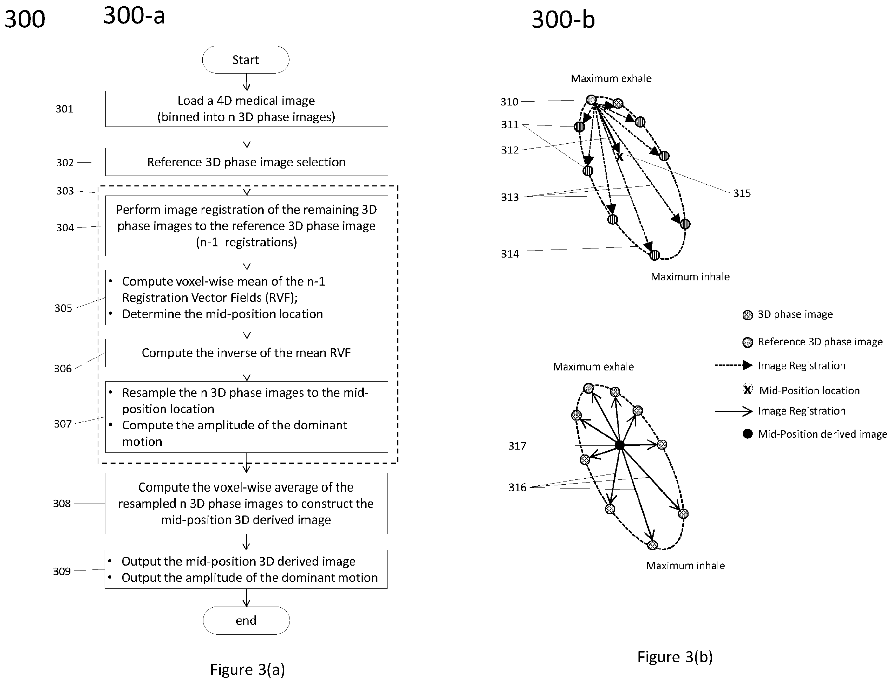

This method is shown in the flow diagram of figure 3. Figure 3 (a) shows a simplified block diagram of a static 3D derived image creation from a 4D medical image with motion compensation using the mid-position method with figure 3 (b) detailing the image registrations relationships. The method starts by loading a 4D medical image 301 consisting of n phase 3D images 311, acquired throughout the breathing cycle 314. First a reference 3D phase image 310 is selected in step 302. Then the core of the motion compensation is performed by the steps 304, 305, 306 and 307 grouped in a single step 303. In 304, the remaining n-1 3D phase images are registered using image registration to the selected reference 3D phase image. This is illustrated by the dashed arrows 313 on figure 3 (b). Then in step 305, a voxel-wise mean of the n-1 registration vector field (RVF) issued from the n-1 registrations is computed. The mean RVF is represented by the arrow 312 starting from the reference phase image 310 and ending up at the mid-position location 315. Once the mid-position location is defined, the inverse of the mean RVF is first computed 306, then all the n 3D phase images are resampled to the mid-position location and the amplitude of the dominant motion is calculated 307. This will require the composition of the inverse of the mean RVF (to go from the mid-position location to the reference 3D phase image) with the reference 3D phase image to phase image registrations 313. The resulting transformations are illustrated with the arrows 316. The mid-position 3D derived image is obtained by a voxel-wise averaging of the resampled n 3D phase images in step 308 and outputs the mid-position 3D derived image and its associated amplitude of the dominant motion 309. As illustrated on Figure 3(b), the mid-position 3D derived image 317 is at the mid-position location 315.

A variant of the mid-position method using a 4D medical image and a different 3D medical image exists. Rather than selecting one of 3D phase images from the 4D medical image as a reference, in step 302, the different 3D image is used to serve as a reference phase image. For instance, a mid-position method using a breath-hold 3D image with or without contrast enhanced as a reference is described in [Nijkamp et al. 2010].

The mid-position location as defined corresponds to the location to which the average deformation from each 3D phase image would be zero. The voxel-wise combination in step 5 is generally considered to be the mean or the median value. The mid-position 3D derived image generation process assumes that all the 3D phase images are equally relevant and therefore creates a motion compensated average image of all 3D phase images. Variations have been proposed that determine poor registrations based on the RVF, to remove 3D phase images that may confound the 3D derived image as a consequence of registration failure or inaccuracy.

Therefore, the mid-position imaging overcomes some of the challenges of respiratory motions, enabling a 3D derived image to be created for planning that is largely free from respiratory motion, and at the same time it provides an estimate of the dominant motion suitable for determining the optimal treatment margin to apply to a target structure such that the tumor is maximally irradiated while minimizing healthy tissue damage. Note however, that these margins only account for the motion determined from the image registration of the 3D phase images, and do not address any secondary motion present in any particular 3D phase image occurring as a result of an alternative physiological cycle, such as the cardiac cycle.

The radiation dose to particular cardiac sub-structures is being increasingly recognized as significant in terms of poor outcomes with respect to cardiac complications and side-effects from radiotherapy [McWilliam 2017, de Almeida 2012, Korreman 2006]. Thus, there is an increasing desire to identify and annotate these sub-structures when performing treatment planning [Vennarini 2013]. However, mid-position imaging does not address cardiac motion, and the resulting average image often has a poorly defined heart incorporating both motion and averaging artefacts. This is because the mid-position approach assumes all 3D phase images of the 4D medical image are equally relevant and will use all 3D phase images of the 4D medical image in creating the mid-position 3D derived image.

As clearly shown in figure 1, the frequency of the cardiac cycle is higher than the breathing cycle, and the two cycles are not synchronized. Returning to figure 1, the cardiac cycle is illustrated by the curve 104, with the cycle alternating between end-diastole, shown by dashed line 105, and end-systole, shown by dashed line 108, over time. The progression of time is shown by line 108. The cardiac signal is not normally recorded during a 4D medical image acquisition but is shown here to illustrate the cardiac signal with respect to the breathing cycle. Nevertheless, such a cardiac signal could be recorded during image acquisition. It can be observed that the position in the cardiac cycle varies during each of the image acquisition windows (109, 110), (111,112), (113,114), and (115,116), which are all binned into the same 3D volumetric image according to the breathing phase. Where the gradient of the cardiac signal is high, such as windows 111-112 and 115-116, the amount of cardiac motion evident in a 2D scan image will be large, since 2D scan image acquisition takes a period time, albeit short, during which cardiac motion can occur. Where the gradient is low (as in windows 109-110, 113-114), the anatomical changes in cardiac sub-structures will be small during that period, thus less motion will be evident in the 2D scan image acquisition. Motion in the image will normally result in artefacts such as image blurring [Dai S and Wu Y 2008].

Since the mid-position 3D derived image averages all the 3D phase images equally, some may contain motion artifacts within the region of the heart - or any other region of the image where there is secondary motion from another physiological cycle not accounted for by the 4D CT acquisition and registration-based motion compensation.

### Problem summary

Thus, there is a need for a method whereby a 3D derived image can be created from a 4D medical image that shows the full anatomical detail of the patient clearly while also enabling a tumor motion estimate to be calculated concurrently to clearly visualizing the anatomy.

According to the invention there is provided a method for creating a motion compensated 3D volumetric derived image from a 4D volumetric medical image comprising the steps of: acquiring a 4D volumetric medical image comprising a plurality of 3D volumetric medical images forming a time-series of 3D volumetric medical images representing phases of a physiological motion cycle; determining dominant motion of each 3D volumetric medical image with respect to a reference location that represents a position relative to the motion cycle; resampling each of the 3D volumetric medical images to the reference location to compensate for the determined dominant motion ; estimating secondary motion in at least one of each motion compensated 3D volumetric medical images or of each of the original 3D volumetric medical images ; and constructing a 3D volumetric derived image by combining data from the motion compensated 3D volumetric images based on the estimated secondary motion.

In a preferred embodiment of the invention estimating the secondary motion comprises determining the secondary motion in each of the resampled phase images and each of the original 3D volumetric phase images.

Preferably, the reference location is one of; the location within the motion cycle of one of the original 3D medical images; an extremum of the motion cycle; a geometric combination of the locations within the motion cycle of the original 3D medical images; the mid-position location of the motion cycle.

Preferably, the method of determining the dominant motion with respect to the reference location comprises determining an image registration of each of the original 3D volumetric images to the reference location.

In an embodiment of the invention determining an image registration of an original 3D volumetric image to the reference location comprises one of; computing a direct image registration between the original 3D volumetric image and a 3D volumetric image representing the reference location; composition of an image registration from the original 3D volumetric image to a second 3D volumetric images with a registration from the second 3D volumetric image to the reference location.

Further preferably, the mid-position location of the motion cycle is determined comprising the following steps; selecting a 3D volumetric image acquired at location within the motion cycle; registering, using image registration, at least one of the original 3D volumetric images to the selected 3D volumetric image; determining a registration by computing the mean of all the image registrations between the selected 3D volumetric image and original 3D volumetric images.

In an embodiment of the invention the estimation of the secondary motion varies spatially within the 3D volumetric medical images.

Further preferably, the 3D volumetric medical images are images of the abdominal or thoracic cavity and the dominant motion is at least one of respiratory motion or cardiac motion.

In a preferred embodiment of the invention the secondary motion is at least one of respiratory motion or cardiac motion.

Further preferably, the image registration is a deformable registration.

Preferably, the modality of the 4D volumetric medical image is a CT image or a MRI image.

In an embodiment of the invention, the selected 3D volumetric image is one of: an original 3D volumetric image forming part of the 4D medical image; a further acquired 3D volumetric medical image,

Preferably, estimating the secondary motion is made according to at least one of; an estimate of blur or sharpness in the 3D volumetric medical image; regional image intensity in the 3D volumetric medical image; a measurement of the difference between the 3D volumetric medical image and a sharpened version of the 3D volumetric medical image; an estimate of the location of a 2D slice of the 3D volumetric medical image within a secondary motion cycle.

Preferably, the estimate of the location within the secondary motion cycle of the 2D slice of the 3D volumetric medical images comprises one or more of: using a measurement device to measure a physiological signal related to the secondary motion; fitting a periodic signal to time ordered data; using automatic image segmentation to determine a change in anatomy; using image processing techniques; using the time stamp of the acquisition of the 2D slice; using the size of an automatically contoured anatomical region within the 2D slice.

Further preferably, estimating the image sharpness is made according to at least one of; image gradient information calculated using differential techniques; image gradient information calculated using frequency techniques; image gradient information calculated using mathematical morphology techniques; assessing the difference from a sharpened version of the same image; assessing the impact of incremental blurring on the original image; or using a machine learning model to estimate the degree of blur/sharpness.

In a preferred embodiment of the invention the estimation of secondary motion is performed on either the resampled 3D volumetric medical image, on the original 3D volumetric medical image, or a combination of both.

Preferably, the estimate of location within the secondary motion cycle of the 2D slice of the 3D volumetric medical image is performed on at least one of the resampled 3D volumetric medical image, or the original 3D volumetric medical image, or a combination of both.

Further preferably, constructing the 3D volumetric derived image based on the estimated secondary motion comprises the steps; selection of data from at least one of the motion compensated 3D volumetric medical images based on the secondary motion estimate; combining the intensity values of the selected data at each voxel; setting the intensity values of the derived 3D volumetric image according to the combined intensity values.

Preferably the method of selecting data from the motion compensated 3D volumetric medical images comprises at least one of; selecting data from the image associated with the lowest secondary motion estimate; selecting data from the N images associated with the lowest secondary motion estimate, where the number N is pre-defined; selecting data from the images associated with a secondary motion estimate lower than a predefined threshold; selecting data from the images associated with a secondary motion estimate lower than an automatically determined threshold.

Preferably the method of selection varies according to the spatial location within the derived 3D volumetric medical image.

In an embodiment of the invention the method of combining the intensity values for the selecting data comprises at least one of; the mean of the intensities; the median of the intensities; a central tendency estimation of the intensities; the mean of the intensities weighted according to their secondary motion estimate; the median of the intensities weighted according to their secondary motion estimate; a central tendency estimation of the intensities weighted according to their secondary motion estimate.

Preferably, selecting the motion corrected 3D volumetric image from which an intensity value is taken varies according to the location within the image.

Further preferably, the reference location is either user defined or pre-set.

According to the invention there is also provided an imaging system for creating a 3D volumetric image from time series volumetric 4D image data according to the methods described above.

### Brief description of the drawings

Further details, aspects and embodiments of the invention will be described, by way of example only, with reference to the drawings. In the drawings, like reference numbers are used to identify like or functionally similar elements. Elements in the figures are illustrated for simplicity and clarity and have not necessarily been drawn to scale.
Figure 1 shows an Illustrative example for respiratory and cardiac cycles during a 4D medical image acquisition;
Figure 2(a) shows Average Intensity Projection prior art method for static 3D derived image creation from a 4D medical image without motion compensation;
Figure 2(b) shows Maximum Intensity Projection prior art method for static 3D derived image creation from a 4D medical image without motion compensation;
Figure 3(a) is a simplified block diagram of a prior art method for static 3D derived image creation from a 4D medical image with motion compensation using the mid-position method;
Figure 3(b) shows schematic representation of the image registrations needed by the mid-position method of the prior art;
Figure 4 shows a simplified block diagram of a static 3D derived image creation from a 4D medical image with motion compensation including construction using post-deformation secondary motion amplitude estimates according to an embodiment of the invention;
Figure 5: shows a simplified bock diagram of a static 3D derived image creation from a 4D medical image with motion compensation including construction using pre-deformation motion amplitude estimates according to an embodiment of the invention;
Figure 6 shows a simplified bock diagram of a static 3D derived image creation from a 4D medical image with motion compensation including construction using combined post-deformation secondary motion amplitude and pre-deformation motion amplitude estimates according to an embodiment of the invention;
Figure 7 shows an example of derived images obtained from three different methods to combine 3D phases images from a 4D medical image into a 3D derived image;

### Invention

The disclosed invention recognizes that the quality of each phase of a binned 4D medical image may not be the same, as assumed by the prior art mid-position method, as a consequence of secondary respiratory and/or cardiac motion. These motion artefacts can be further reduced in the 3D derived image output by determining the best 3D phase images or 2D scan image slices or phases where the motion artefacts are reduced. The combination of medical images acquired at locations with a respiratory and/or cardiac cycle with lower motion artefacts will lead to a 3D derived image with reduced motion artefacts. However, the tumour motion estimate calculated during computation of the mid-position image can still be determined from the registration of all 3D phase images.

In an embodiment of the invention the method for creating a motion compensated 3D volumetric derived image from a time-series of volumetric 4D medical images initially proceeds according to the method previously defined for mid-position imaging. That is the method includes the steps of acquiring a plurality of 3D volumetric medical images forming a time-series of volumetric medical images, representing phases of a physiological motion cycle; registering one or more of the acquired 3D volumetric medical images to a selected reference location of a dominant motion cycle. In an embodiment of the invention the method then proceeds by resampling each 3D volumetric medical image to a reference location to compensate for the dominant motion in the motion cycle using the information from the image registrations; estimating the secondary motion in at least one of each motion compensated 3D volumetric medical images or each of the original 3D volumetric medical images ; and constructing a 3D volumetric derived image by combining data from the motion corrected 3D volumetric images based on the estimated secondary motion for the 3D volumetric images. In a preferred embodiment of the invention, constructing the 3D volumetric derived image based on the estimated secondary motion comprises the steps; selection of data from at least one of the motion compensated 3D volumetric medical images based on the secondary motion estimate; combining the intensity values of the selected data at each voxel; setting the intensity values of the derived 3D volumetric image according to the combined intensity values. Preferably, the method of selecting data from the motion compensated 3D volumetric medical images comprises at least one of; selecting data from the image associated with the lowest secondary motion estimate; selecting data from the N images associated with the lowest secondary motion estimate, where the number N is pre-defined; selecting data from the images associated with a secondary motion estimate lower than a predefined threshold; selecting data from the images associated with a secondary motion estimate lower than an automatically determined threshold. In an embodiment of the invention the method of selection varies according to the spatial location within the derived 3D volumetric medical image.

In a further preferred embodiment of the invention, the method of combining the intensity values for the selecting data comprises at least one of; the mean of the intensities; the median of the intensities; a central tendency estimation of the intensities; the mean of the intensities weighted according to their secondary motion estimate; the median of the intensities weighted according to their secondary motion estimate; a central tendency estimation of the intensities weighted according to their secondary motion estimate. In an example of the invention, selecting the motion corrected 3D volumetric image from which an intensity value is taken varies according to the location within the image.

In a preferred embodiment of the invention estimating the secondary motion comprises determining the secondary motion in each of the motion compensated 3D volumetric images and each of the original 3D volumetric medical images. In an embodiment of the invention estimating the secondary motion is made according to at least one of; an estimate of blur or sharpness in the 3D volumetric medical image; regional image intensity in the 3D volumetric medical image; a measurement of the difference between the 3D volumetric medical image and a sharpened version of the 3D volumetric medical image; an estimate of the location of a 2D slice of the 3D volumetric medical image within a secondary motion cycle. Preferably the image registration is a deformable registration, although other registrations may alternatively be used. Preferably, a mid-position location in the motion cycle used, where this is defined to correspond to the location to which the average deformation from each 3D phase image would be zero. However, in alternative embodiments of the invention other positions relative to the motion cycle may be used. However, rather than defining the 3D derived image as the average of all 3D phase images, or the average of well-registered 3D phase images, as in the prior art, this invention is to use only the data from the 2D scan images, or the 3D phases images composited from them, with a low level of secondary motion.

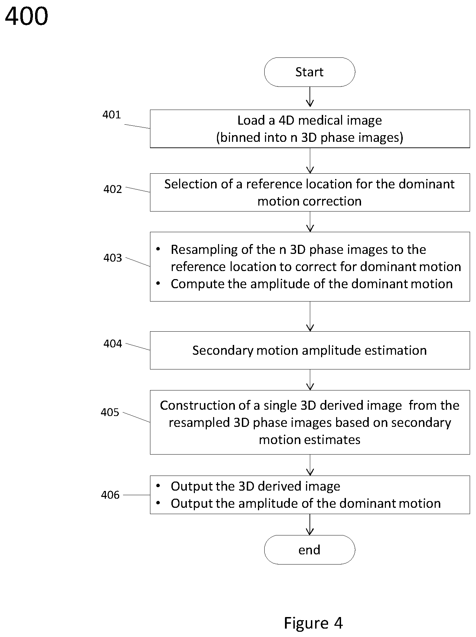

Figure 4, shows a simplified block diagram of the method for creation of a static 3D derived image from a 4D medical image with motion compensation including construction of a single 3D derived image using post-deformation secondary motion amplitude estimates, as used in an embodiment of this invention. In a preferred embodiment of the invention, a plurality of 3D volumetric medical images forming a time series of volumetric medical images representing the phases of a physiological motion cycle are acquired (not shown in the figure). In a preferred embodiment of the invention the acquired 3D volumetric medical images are CT images or MRI images. Preferably, the images are images of the thoracic or abdominal cavity, and the physiological motion cycles and dominant motion is at least one of respiratory motion or cardiac motion. These acquired images are registered to a selected location of a dominant motion cycle. Preferably, the image registration method is a deformable registration.

As shown, the method starts with loading a 4D medical image 401 consisting of n 3D volumetric medical images comprising 2D scan images acquired at specific phases of the motion cycle (respiratory and/or cardiac motion) so that a motion compensated 3D volumetric derived image can be created. Then a reference location relative the motion cycle is selected for the dominant motion correction in step 402. In an embodiment of the invention the reference location is one of; the location within the motion cycle of one of the original 3D medical images; an extremum of the motion cycle; a geometric combination of the locations within the motion cycle of the original 3D medical images; the mid-position location of the motion cycle. In a preferred embodiment of the invention the method of determining the dominant motion with respect to the reference location comprises determining an image registration of each of the original 3D volumetric images to the reference location. Preferably, the reference location is user defined or pre-set. In a further preferred embodiment of the invention determining an image registration of an original 3D volumetric image to the reference location comprises one of; computing a direct image registration between the original 3D volumetric image and a 3D volumetric image representing the reference location; composition of an image registration from the original 3D volumetric image to a second 3D volumetric images with a registration from the second 3D volumetric image to the reference location.

Then motion compensation for all 3D volumetric medical images of the 4D medical image is performed in step 403 by resampling all the n 3D phase images to a selected location in the motion cycle. Preferably, this will be the mid-position location, but other locations relative to the motion cycle may also be used. In a preferred embodiment of the invention, the mid-position location of the motion cycle is determined comprising the following steps; selecting a 3D volumetric image acquired at location within the motion cycle; registering, using image registration, at least one of the original 3D volumetric images to the selected 3D volumetric image; determining a registration by computing the mean of all the image registrations between the selected 3D volumetric image and original 3D volumetric images. Preferably, the selected 3D volumetric image is one of: an original 3D volumetric image forming part of the 4D medical image or a further acquired 3D volumetric medical image. The associated amplitude of the dominant motion is calculated. In a preferred embodiment of the invention the resampling of each 3D phase image is done by calculating a deformation of each 3D phase image and using the calculated deformation to define a common reference space. Preferably, the deformable registration is an average deformation, and the common reference space is defined from the average deformation with respect to the reference location of the motion cycle.

The required processing in step 403 may be performed in the manner previously detailed in step 303 of figure 3, although other methods may also be possible.

After all the 3D phase images are compensated for the dominant motion (respiratory or cardiac motion), a secondary motion amplitude estimation is performed in step 404. Preferably, the estimation of secondary motion comprises estimating the secondary motion in each of the resampled 3D phase images and each of the original 3D volumetric medical images. In an embodiment of the invention, the estimation of secondary motion varies spatially within the volumetric images. This information is then used in step 405 in the construction of a single 3D volumetric derived image by combining data from the motion corrected 3D volumetric images based on the estimated secondary motion for the 3D volumetric images. The volumetric derived image is output in step 406, together with the associated amplitude of the dominant motion.

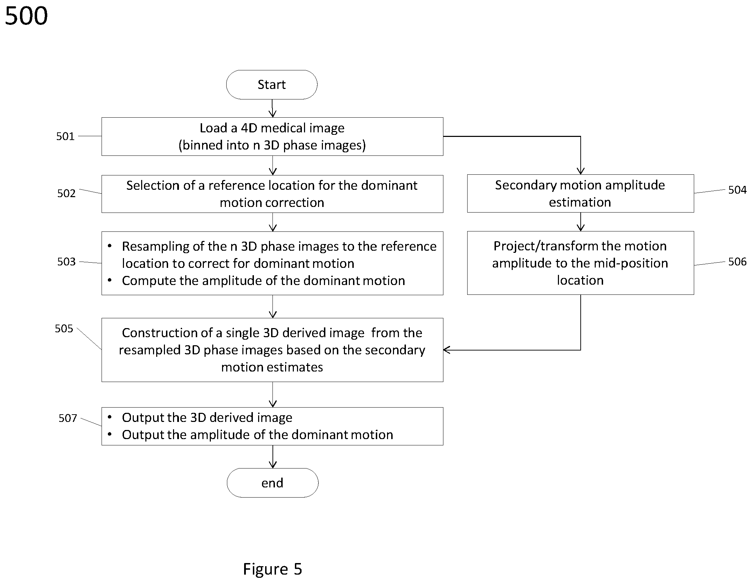

Figure 5: shows a simplified block diagram of an embodiment of the invention showing the method of creating a static 3D derived image from a 4D medical image with motion compensation including construction using pre-deformation motion amplitude estimates, as used in an embodiment of this invention. In this embodiment of the invention, the data loading step 501, the reference location selection step 502 and the motion compensation and dominant motion amplitude estimate step 503 are similar to the corresponding steps as shown in Figure 4. The construction of the 3D derived image in step 505, however, uses motion amplitude estimation obtained on the original scan image or phase image data 504, i.e before the dominant motion correction. Thus, the amplitude estimation is first projected or transformed to the mid-position location 506, before its use in the image construction step 505 to output a single 3D derived image and its associated dominant motion amplitude in step 507.

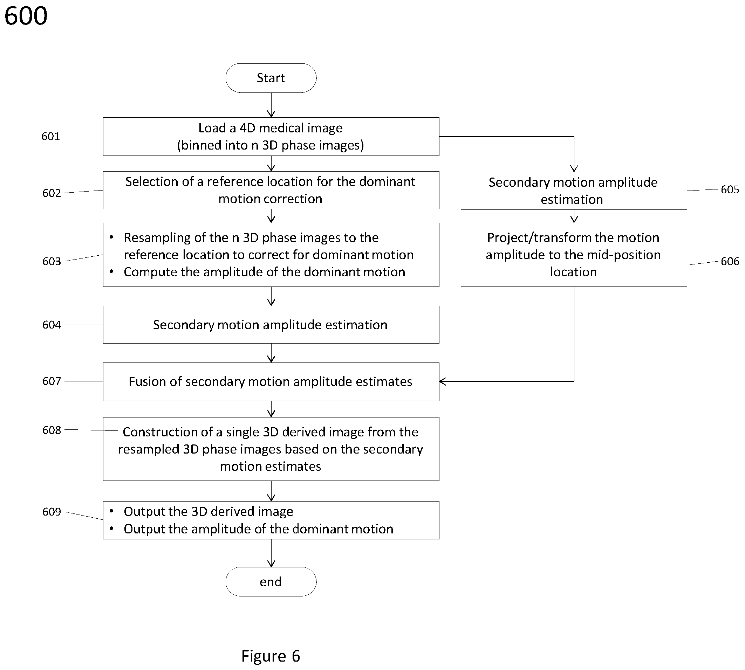

Figure 6 shows a simplified block diagram of an embodiment of the invention for the method of creation of a static 3D derived image from a 4D medical image with motion compensation including construction using combined post-deformation secondary motion amplitude and pre-deformation secondary motion amplitude estimates, as used in an embodiment of this invention. In this embodiment of the invention, the data loading step 601, the reference location selection step 602 and the motion compensation and dominant motion amplitude calculation step 603 are similar to the corresponding steps as shown in Figure 4. In this embodiment, a secondary motion amplitude estimation 604 on the corrected phase is fused 607 with secondary motion amplitude estimates on the original data 605. The combined motion amplitude estimate is then used in the image construction step 608 to output a single 3D derived image and its associated dominant motion amplitude in step 609.

This invention also provides an imaging system for creating a 3D volumetric image from time series volumetric 4D image data, according to the various methods described above.

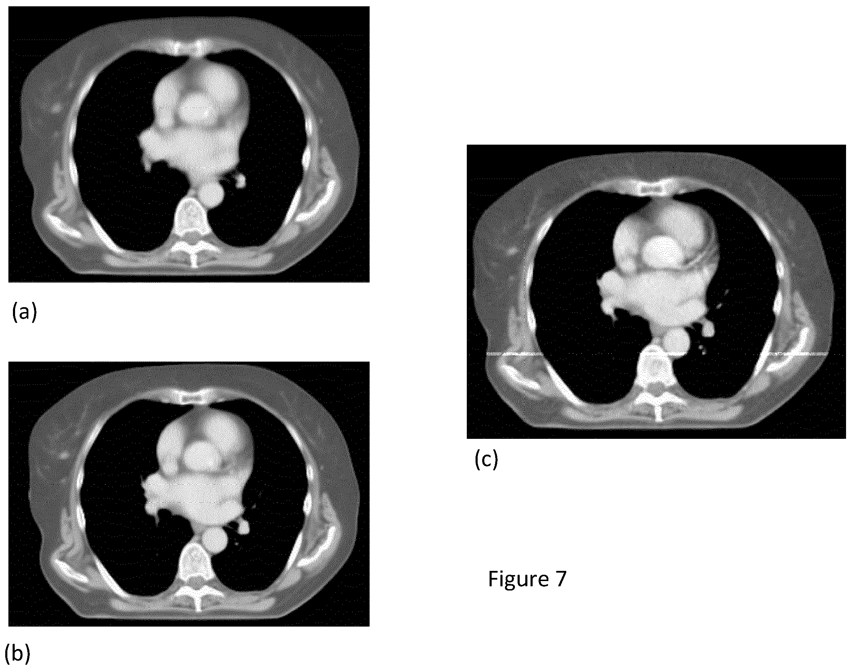

Figure 7 provides examples of 3D derived images. Figure 7(a) shows a slice from a 3D derived image that is created using the average intensity projection method of the prior art, figure 7(b) shows a slice from a 3D derived image that is created using the mid-position scan (respiration compensated) method of the prior art. Figure 7(c) shows a slice from a 3D derived image that is created using the method of this invention. As shown, the features in the heart region are much sharper in the image of figure 7(c).

Taking a typical example for radiotherapy planning, where the 4D medical image is binned according to a respiratory motion signal, the mid-position method creates a derived image that has been compensated for this respiratory motion. However, cardiac motion remains, and there has not been any compensation in the derived image for the cardiac motion. Therefore, this secondary motion leads to a blurred appearance of the heart in the derived image or any anatomical region that is moving because of the cardiac motion. Images acquired at some locations of the cardiac cycle will have a better-defined heart, since the heart typically remains at end-diastole for a longer period that any other point in the cycle and is moving slower, than other points in the cycle where the heart is moving rapidly. Thus, by only selecting and combining data from 2D scan images or 3D phase images in which the heart appears quasi-static when creating the 3D derived image, a respiratory motion compensated and near cardiac motion free image can be generated with a clearly defined heart.

The same approach could be taken if the 4D medical image is binned according to a different motion signal. In some embodiments of the invention the dominant motion cycle could be the respiratory cycle and the secondary motion cycle could be cardiac motion. In other embodiments of the invention the dominant motion cycle could be the cardiac cycle and the secondary motion cycle could be the respiratory cycle. It's presence in the images could be minimized by selecting the scan or phase images with the least motion artefacts, which would generally correspond to the maximum exhale of the respiratory cycle.

Various techniques can be used to determine the amount of secondary respiratory and/or cardiac motion; One approach would be to determine the location within the physiological cycle that is resulting in secondary motion using a measurement device. For instance, if the 4D medical image is binned according to the respiratory cycle, the cardiac signal can also be measured during the period of acquisition using an electrocardiogram. The knowledge that there are particular stages of the physiological cycle that have lower motion would allow this signal to be used as a surrogate estimate of motion.

Another approach is to consider the sharpness of an image. In the embodiments of the invention as described, image sharpness and image blur are related concepts. Specifically, maximizing image sharpness is the same as minimizing image blur. Thus, any reference in the description of the invention to sharpness or blur should be interpreted to include its alternative. Respiratory and /or cardiac motion during the acquisition of a scan image results in blurring artefacts in the image. Therefore, images with reduced motion artefacts will appear sharper with reduced blurring and therefore have greater image contrast between different neighboring tissues. Basic image processing techniques such as calculation of an image intensity gradient can be used to estimate image sharpness. Thus, the image sharpness can be used as a surrogate of secondary motion within the image.

In a preferred embodiment of the invention selecting the sharpest image is made according to at least one of; image gradient information calculated using differential techniques; image gradient information calculated using frequency techniques; image gradient information calculated mathematical morphology techniques; assessing the difference from a sharpened version of the same image; assessing the impact of incremental blurring on the original image; of using a machine learning model to estimate the degree of blur/sharpness.

Thus, in an embodiment of the invention, data from 2D scan images or 3D phase images may be selected based on the magnitude of image gradients, or parameters that are derived from them, such as the Laplacian, or observed within or between adjacent tissues. Image gradients may be estimated using simple discrete difference methods, or more complex approaches such a phase-congruency based approaches, or non-linear techniques to estimate the image gradient such as grey level mathematical morphology. A wide variety of techniques are known to those skilled in the art to estimate the degree of motion from the blur in an image e.g. [Tiwari et al 2013]. Note, in some applications image blur may also result from a lack of focus - in tomographic imaging the equivalent is inaccurate image reconstruction from the raw projection data. Such focal blur will be similar for all 3D phase images since the acquisition and reconstruction of the 4D medical image is using the same hardware and reconstruction algorithm for all 2D scan images.

Frequency based techniques can also be used, as a sharper image should have more high frequency content. This can be used in combination with denoising techniques that reduces the high frequency content of the noise but preserves the edges, thus reducing the amount of the blur or increasing the sharpness the image. Such techniques include for example, bilateral filtering, anisotropic diffusion, or non-local means. Image enhancement techniques can also be used to artificially sharpen the original phase. This can for example be achieved using techniques to reverse the heat equation [Buades et al 2006], mathematical morphology filtering or any deblurring technique.

The difference of the original 3D phase image or 2D scan image from its sharpened version may then be used to determine the degree of respiratory or cardiac motion - the greater the difference between the images, the softer the original medical image, and therefore the greater the likelihood of respiratory or cardiac motion artefacts. Alternatively, the amount of impact that incremental image blurring has on the medical image can be used to estimate the sharpness of the original medical image. Higher contrast boundaries will be more greatly affected by blurring operations. Direct or indirect estimation of the amount of the image sharpness/blur can also be performed using machine learning techniques [Sun et al 2015, Xiao et al. 2021]. Note that these techniques regarded the blur detection as an image segmentation problem and thus can provide a pixel-wise estimation.

The amount of respiratory and/or cardiac motion artefact can also be estimated by determining the temporary location of a 2D scan image within the motion cycle from the image. In a preferred embodiment of the invention, an estimate of location of the 2D image within the motion cycle is made according to at least one of; regional image intensity in the image; the time-stamp of 2D image acquisition; using the size of an automatically contoured anatomical region within the 2D image; a periodic model fit to estimates from more than on image; the measurement of a physical signal. In a further embodiment of the invention the estimate of the location within the secondary physiological motion cycle of the 2D slice of the 3D volumetric medical image is performed on at least one of the deformed 3D volumetric image, or the original undeformed 3D volumetric image, or a combination of both.

The various anatomic motion cycles have points within them where the motion is low, and therefore selection of voxels to include in the 3D derived image can be done by determining which 2D scan images belong to these points in the cycle. Various approaches to determine the location in the cycle can made; For example, where contrast media is used to enhance contrast of the blood pool within a CT or MRI image, the average intensity value within the region around the heart increases and decreases as the heart beats. This is because the heart expands and contracts as it beats, therefore changing the amount of blood in the region being observed. The maxima and minima of this signal correspond to end-diastole and end-systole, where cardiac motion is lowest. Similarly, automatic segmentation methods, such as those using machine learning, can be used to estimate the organ size and therefore determine the location in the anatomical motion cycle.

To further refine the location within the motion cycle, the time-stamp from the 2D scan images may be used. On modern imaging equipment the time-stamp is recorded for the acquisition time of each 2D scan image used to create the 3D phase images. Thus, the full 4D medical image sequence can be decomposed into time-ordered 2D scan images. A subset of these will contain relevant slices, such as those containing the heart when considering cardiac motion. From this subset, the point in motion cycle can be estimated using image processes techniques, as described previously. An appropriate periodic signal, for example a sinusoid, may be fit to these estimates. The regular nature of these anatomical cycles means that the estimate of the location within the cycle can be better estimated by fitting a periodic signal to mitigate the effects of signal noise or incorrect estimates on some 2D scan images. Preferably, in an example of the invention, the estimate of the location within the secondary motion cycle of the 2D slice of the 3D volumetric medical images comprises one or more of: using a measurement device to measure a physiological signal relating to the secondary motion; fitting a periodic signal to time ordered data; using automatic image segmentation to determine a change in anatomy; using image processing techniques; using the time stamp of the acquisition of the 2D slice; using the size of an automatically contoured anatomical region within the 2D slice. In an embodiment of the invention, the estimation of secondary motion is performed on either the resampled 3D volumetric medical image, on the original 3D volumetric medical image, or a combination of both. In a further embodiment of the invention, the estimate of location within the secondary motion cycle of the 2D slice of the 3D volumetric medical image is performed on at least one of the resampled 3D volumetric medical image, or the original 3D volumetric medical image, or a combination of both.

This estimated periodic signal can then be used to determine the location of each 2D scan image within the anatomical motion cycle and used as a surrogate of the secondary motion.

Having determined or estimated the secondary motion within each 2D scan image for all images within the 4D medical image, the 3D derived image must be constructed by selecting or weighting data from the 3D medical image or images, or 2D scan image or images, with the least estimated secondary motion. The images may be selected as those with the lowest estimated secondary motion, or alternatively may be selected as those with an estimated secondary motion below a threshold value. This threshold value may be predetermined. Alternatively, this threshold value may be automatically determined; for example, the threshold may be set to relative to the lowest motion estimate or as a percentile of the motion estimates. However, at all spatial locations for the 3D derived image data from at least one image must be selected.

Data from the images with the least estimated secondary motion may be selected, or weighted, on a global (whole volume basis). However, since 4D binning is performed (for CT) on a 2D slice-by-slice basis, there may be differing degrees of motion for different 2D scan images within a 3D phase image. Thus, selection or weighting of the data to include in the final 3D derived image may vary according to the slice in the image. Furthermore, the degree of motion may vary spatially within a 2D scan image or within the 3D phase image, thus selection or weighting of data with low motion may be performed patch-wise at different locations within the image.

The data may be selected or weighted on the basis of the sharpness of the original medical image, prior to deformation to the common reference space. This approach gives an estimate of the degree of motion in the original medical image, undistorted by the dominant motion compensation performed by image registration. However, deformation may appear to introduce motion artifacts, particularly if the reference 3D phase image chosen for registration has artifacts. Therefore, an alternative is to perform selection/weighting on the deformed medical images, giving an estimate of the amount of motion artefact apparent in each 3D phase image after the deformation. Alternatively, a combination of sharpness measures from both the undeformed and deformed images can be used to select/weight data from medical images which are both originally low in motion artefacts and have not had any introduced by the registration process.

In some regions, secondary motion may be small, and greater benefit to the 3D image quality may be derived by combining a greater number of motion-corrected 3D phase images. Thus, it may be necessary to define different rules for data selecting/weighting to include in the 3D derived image construction process according to the location within the image. This can be achieved by automatically determining the location in the image by using machine learning methods for example. Alternatively, rules may be defined according to anatomy, and methods such as automatic image segmentation may be used to automatically define anatomical regions and thereby determine which selection or weighting rules to apply.

Construction always occurs from the 4D medical image data after the deformation, meaning after the dominant motion compensation. Construction occurs from the 3D phase images or 2D scan images for each region of the 3D image, whereby the region can be considered to be the whole 3D phase image, a 2D scan image, a local patch within a medical image, an anatomic region, or even a single voxel, based on the motion estimate. This motion estimate can have been determined slice-by-slice within the 3D phase images (i.e. the 2D scan images), locally within patches of the 3D phase images, or within an anatomic region within each 3D phase image, or even at each voxel.

One approach to constructing the 3D derived image, is that for each region, image data is selected from the medical image with the lowest motion estimate. This image data is used directly within the 3D derived image.

An alternative approach is that for each region a subset of the medical images with the 4D medical image are selected that have the lowest motion amplitude estimates. The image data from these regions is then combined in the 3D derived image, for instance either by taking an average intensity for each voxel, or by taking a weighted average whereby the contribution of data from each medical image is weighted according to the motion amplitude estimate with the medical image receiving the lowest estimated motion amplitude having the highest contributing weight.

The selection of a subset of image data for each region can be made by selecting a fixed number of medical images with the lowest motion estimate, where the fixed number is a pre-defined or user-provided setting. Alternatively, the selection of a subset of image data for each region can be made by selecting any number of medical images where the motion amplitude estimate is below a pre-defined or user-provided value.

For all regions in the 3D derived image, image data is taken, whether selected or combined from multiple phases from the 3D phase images or 2D scan images. The combination operation can be either the mean or the median. The weighted combination can be either the weighted mean or the weighted median or any central tendency estimator [Upton and Cook 2008].

The present invention has been described with reference to the accompanying drawings. However, it will be appreciated that the present invention is not limited to the specific examples herein described and as illustrated in the accompanying drawings. Furthermore, because the illustrated embodiments of the present invention may for the most part, be implemented using electronic components and circuits known to those skilled in the art, details will not be explained in any greater extent than that considered necessary as illustrated above, for the understanding and appreciation of the underlying concepts of the present invention and in order not to obfuscate or distract from the teachings of the present invention.

The invention may be implemented in a computer program for running on a computer system, at least including code portions for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system. Therefore, some examples describe a non-transitory computer program product having executable program code stored therein for automated contouring of cone-beam CT images.

The computer program may be stored internally on a tangible and non-transitory computer readable storage medium or transmitted to the computer system via a computer readable transmission medium. All or some of the computer program may be provided on computer readable media permanently, removably or remotely coupled to an information processing system. The tangible and non-transitory computer readable media may include, for example and without limitation, any number of the following: magnetic storage media including disk and tape storage media; optical storage media such as compact disk media (e.g., CD-ROM, CD-R, etc.) and digital video disk storage media; non-volatile memory storage media including semiconductor-based memory units such as FLASH memory, EEPROM, EPROM, ROM; ferromagnetic digital memories; MRAM; volatile storage media including registers, buffers or caches, main memory, RAM, etc.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims and that the claims are not limited to the specific examples described above.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures can be implemented which achieve the same functionality.

Any arrangement of components to achieve the same functionality is effectively 'associated' such that the desired functionality is achieved. Hence, any two components herein combined to achieve a particular functionality can be seen as 'associated with' each other such that the desired functionality is achieved, irrespective of architectures or intermediary components.

Likewise, any two components so associated can also be viewed as being 'operably connected,' or 'operably coupled,' to each other to achieve the desired functionality.

Furthermore, those skilled in the art will recognize that boundaries between the above described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms 'a' or 'an,' as used herein, are defined as one or more than one. Also, the use of introductory phrases such as 'at least one' and 'one or more' in the claims should not be construed to imply that the introduction of another claim element by the indefinite articles 'a' or 'an' limits any particular claim containing such introduced claim element to inventions containing only one such element, even when the same claim includes the introductory phrases 'one or more' or 'at least one' and indefinite articles such as 'a' or 'an.' The same holds true for the use of definite articles. Unless stated otherwise, terms such as 'first' and 'second' are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

### References

Brock, K. K., Mutic, S., McNutt, T. R., Li, H., & Kessler, M. L. (2017). Use of image registration and fusion algorithms and techniques in radiotherapy: Report of the AAPM Radiation Therapy Committee Task Group No. 132: Report. Medical Physics, 44(7), e43-e76. https://doi.org/10.1002/mp.12256
Buades, A., Coll, B., & Morel, J. (2006). Image enhancement by non-local reverse heat equation. Preprint CMLA.
Dai S, Wu Y. Motion from blur. In 2008 IEEE Conference on Computer Vision and Pattern Recognition 2008 Jun 23 (pp. 1-8). IEEE.
de Almeida CE, Fournier-Bidoz N, Massabeau C, Mazal A, Canary PC, Kuroki IR, Campana F, Fourquet A, Kirova YM. Potential benefits of using cardiac gated images to reduce the dose to the left anterior descending coronary during radiotherapy of left breast and internal mammary nodes. Cancer/Radiothérapie. 2012 Feb 1;16(1):44-51.
Keall, P. J., Mageras, G. S., Balter, J. M., Emery, R. S., Forster, K. M., Jiang, S. B., Kapatoes, J. M., Low, D. A., Murphy, M. J., Murray, B. R., Ramsey, C. R., Van Herk, M. B., Vedam, S. S., Wong, J. W., & Yorke, E. (2006). The management of respiratory motion in radiation oncology report of AAPM Task Group 76. Medical Physics, 33(10), 3874-3900. https://doi.org/10.1118/1.2349696
Korreman SS, Pedersen AN, Josipović M, Aarup LR, Juhler-Nøttrup T, Specht L, Nyström H. Cardiac and pulmonary complication probabilities for breast cancer patients after routine end-inspiration gated radiotherapy. Radiotherapy and oncology. 2006 Aug 1;80(2):257-62.
Maintz JA, Viergever MA. A survey of medical image registration. Medical image analysis. 1998 Mar 1;2(1):1-36.
McWilliam A, Kennedy J, Hodgson C, Osorio EV, Faivre-Finn C, van Herk M. Radiation dose to heart base linked with poorer survival in lung cancer patients. European Journal of Cancer. 2017 Nov 1;85:106-13.
Murphy, K., Van Ginneken, B., Reinhardt, J. M., Kabus, S., Ding, K., Deng, X., Cao, K., Du, K., Christensen, G. E., Garcia, V., Vercauteren, T., Ayache, N., Commowick, O., Malandain, G., Glocker, B., Paragios, N., Navab, N., Gorbunova, V., Sporring, J., ... Pluim, J. P. W. (2011). Evaluation of registration methods on thoracic CT: The EMPIRE10 challenge. IEEE Transactions on Medical Imaging, 30(11), 1901-1920. https://doi.org/10.11109/TMI.2011.2158349
Nijkamp, J., Rit, S., Herk, M. Van, & Sonke, J. (2010). Utilization of 4D-CT and contrast enhanced expiration breath-hold CT for 3D treatment planning of lung. Proceedings of the XVIth ICCR, 3-6. https://hal.archives-ouvertes.fr/hal-01967311
Sun, J., Cao, W., Xu, Z., & Ponce, J. (2015). Learning a convolutional neural network for non-uniform motion blur removal. Proceedings of the IEEE Computer Society Conference on Computer Vision and Pattern Recognition. https://doi.org/10.1109/CVPR.2015.7298677
Tiwari S, Shukla VP, Singh AK, Biradar SR. Review of motion blur estimation techniques. Journal of Image and Graphics. 2013 Dec;1(4):176-84.
Upton, G.; Cook, I. (2008) Oxford Dictionary of Statistics, OUP ISBN 978-0-19-954145-4
Vennarini S, Fournier-Bidoz N, Aristei C, De Almeida CE, Servois V, Campana F, Mosseri V, Fourquet A, Kirova YM. Visualisation of the left anterior descending coronary artery on CT images used for breast radiotherapy planning. The British journal of radiology. 2013 May;86(1025):20120643.
Xiao, X., Yang, F., & Sadovnik, A. (2021). Msdu-net: A multi-scale dilated u-net for blur detection. Sensors, 21(5), 1-13. https://doi.org/10.3390/s21051873
Wolthaus, J. W.; Sonke, J.-J.; van Herk, M.; Belderbos, J. S.; Rossi, M. M.; Lebesque, J. V. & Damen, E. M. Comparison of different strategies to use four-dimensional computed tomography in treatment planning for lung cancer patients, International Journal of Radiation Oncology* Biology* Physics, Elsevier, 2008 , 70 , 1229-1238

## Claims

1. A computer implemented method (400, 500, 600) for creating a motion compensated 3D volumetric derived image from a 4D volumetric medical image comprising the steps of:
acquiring a 4D volumetric medical image (401, 501, 601) comprising a plurality of 3D volumetric medical images forming a time-series of 3D volumetric medical images representing phases of a physiological motion cycle;
determining dominant motion (403, 503, 603) of each 3D volumetric medical image with respect to a reference location that represents a position relative to the motion cycle;
resampling each of the 3D volumetric medical images to the reference location to compensate for the determined dominant motion ;
estimating secondary motion (404, 504, 604) in at least one of each motion compensated 3D volumetric medical images or of each of the original 3D volumetric medical images ;
and
constructing a 3D volumetric derived image (405, 505, 605) by combining data from the motion compensated 3D volumetric images based on the estimated secondary motion.

2. A method (400, 500, 600) as claimed in claim 1 wherein estimating the secondary motion (404, 504, 604) comprises determining the secondary motion in each of the motion compensated 3D volumetric medical images and each of the original 3D volumetric medical images.

3. A method (400, 500, 600) as claimed in claim 1 or 2 wherein the reference location is one of;
the location within the motion cycle of one of the original 3D medical images;
an extremum of the motion cycle;
a geometric combination of the locations within the motion cycle of the original 3D medical images;
the mid-position location of the motion cycle.

4. A method (400, 500, 600) as claimed in claim 1 or 2 wherein the method of determining the dominant motion (403, 503, 603) with respect to the reference location comprises determining an image registration of each of the original 3D volumetric images to the reference location.

5. A method (400, 500, 600) as claimed in claim 4 wherein determining an image registration of an original 3D volumetric image to the reference location comprises one of;
computing a direct image registration between the original 3D volumetric image and a 3D volumetric image representing the reference location;
composition of an image registration from the original 3D volumetric image to a second 3D volumetric images with a registration from the second 3D volumetric image to the reference location.

6. A method (400, 500, 600) as claimed in claim 3 where the mid-position location of the motion cycle is determined comprising the following steps;
selecting a 3D volumetric image acquired at location within the motion cycle;
registering, using image registration, at least one of the original 3D volumetric images to the selected 3D volumetric image;
determining a registration by computing the mean of all the image registrations between the selected 3D volumetric image and original 3D volumetric images.

7. A method (400, 500, 600) as claimed in any preceding claim wherein the estimation of the secondary motion (404, 504, 604) varies spatially within the 3D volumetric medical images.

8. A method (400, 500, 600) as claimed in any preceding claim wherein the 3D volumetric medical images are images of the abdominal or thoracic cavity and the dominant motion is at least one of respiratory motion and cardiac motion, and the secondary motion is at least one of respiratory motion and cardiac motion.

9. A method (400, 500, 600) as claimed in any preceding claim wherein estimating the secondary motion (404, 504, 604) is made according to at least one of;
an estimate of blur or sharpness in the 3D volumetric medical image;
regional image intensity in the 3D volumetric medical image;
a measurement of the difference between the 3D volumetric medical image and a sharpened version of the 3D volumetric medical image;
an estimate of the location of a 2D slice of the 3D volumetric medical image within a secondary motion cycle.

10. A method (400, 500, 600) as claimed in claim 9 wherein the estimate of the location within the secondary motion cycle of the 2D slice of the 3D volumetric medical images comprises one or more of:
using a measurement device to measure a physiological signal related to the secondary motion;
fitting a periodic signal to time ordered data;
using automatic image segmentation to determine a change in anatomy;
using image processing techniques
using the time stamp of the acquisition of the 2D slice;
using the size of an automatically contoured anatomical region within the 2D slice.

11. A method (400, 500, 600) as claimed in claim 9 wherein estimating the image sharpness is made according to at least one of;
image gradient information calculated using differential techniques;
image gradient information calculated using frequency techniques;
image gradient information calculated using mathematical morphology techniques;
assessing the difference from a sharpened version of the same image;
assessing the impact of incremental blurring on the original image;
using a machine learning model to estimate the degree of blur/sharpness.

12. A method (400, 500, 600) as claimed in any preceding claim wherein constructing the 3D volumetric derived image based on the estimated secondary motion comprises the steps;
selection of data from at least one of the motion compensated 3D volumetric medical images based on the secondary motion estimate;
combining the intensity values of the selected data at each voxel;
setting the intensity values of the derived 3D volumetric image according to the combined intensity values.

13. A method (400, 500, 600) as claimed in claim 12 wherein the method of selecting data from the motion compensated 3D volumetric medical images comprises at least one of;
selecting data from the image associated with the lowest secondary motion estimate;
selecting data from the N images associated with the lowest secondary motion estimate, where the number N is pre-defined;
selecting data from the images associated with a secondary motion estimate lower than a predefined threshold;
selecting data from the images associated with a secondary motion estimate lower than an automatically determined threshold; and wherein the method of selection varies according to the spatial location within the derived 3D volumetric medical image

14. A method (400, 500, 600) as claimed in claim 12 or 13 wherein the method of combining the intensity values for the selecting data comprises at least one of;
the mean of the intensities;
the median of the intensities;
a central tendency estimation of the intensities;
the mean of the intensities weighted according to their secondary motion estimate;
the median of the intensities weighted according to their secondary motion estimate;
a central tendency estimation of the intensities weighted according to their secondary motion estimate.

15. An imaging system for creating a 3D volumetric image from time series volumetric 4D image data comprising means for carrying out method of claims 1-14.

## Patentansprüche

1. Computerimplementiertes Verfahren (400, 500, 600) zum Erzeugen eines bewegungskompensierten volumetrischen abgeleiteten 3D-Bildes aus einem volumetrischen medizinischen 4D-Bild, umfassend die folgenden Schritte:
Erfassen eines volumetrischen medizinischen 4D-Bildes (401, 501, 601), umfassend eine Vielzahl von volumetrischen medizinischen 3D-Bildern, die eine Zeitreihe von volumetrischen medizinischen 3D-Bildern bilden, die Phasen eines physiologischen Bewegungszyklus repräsentieren;
Bestimmen einer dominanten Bewegung (403, 503, 603) jedes volumetrischen medizinischen 3D-Bildes mit Bezug zu einem Referenzort, der eine Position relativ zu dem Bewegungszyklus repräsentiert;
erneutes Abtasten jedes der volumetrischen medizinischen 3D-Bilder zu dem Referenzort, um die bestimmte dominante Bewegung zu kompensieren;
Schätzen der Sekundärbewegung (404, 504, 604) in mindestens einem von jedem bewegungskompensierten volumetrischen medizinischen 3D-Bild oder von jedem der ursprünglichen volumetrischen medizinischen 3D-Bilder;
und
Konstruieren eines volumetrischen abgeleiteten 3D-Bildes (405, 505, 605) durch Kombinieren von Daten aus den bewegungskompensierten volumetrischen 3D-Bildern basierend auf der geschätzten Sekundärbewegung.

2. Verfahren (400, 500, 600) nach Anspruch 1, wobei Schätzen der Sekundärbewegung (404, 504, 604) Bestimmen der Sekundärbewegung in jedem der bewegungskompensierten volumetrischen medizinischen 3D-Bilder und jedem der ursprünglichen volumetrischen 3D-medizinischen Bilder umfasst.

3. Verfahren (400, 500, 600) nach Anspruch 1 oder 2, wobei der Referenzort einer von Folgendem ist:
dem Ort innerhalb des Bewegungszyklus eines der ursprünglichen medizinischen 3D-Bilder;
einem Extremwert des Bewegungszyklus;
einer geometrischen Kombination der Orte innerhalb des Bewegungszyklus der ursprünglichen medizinischen 3D-Bilder;
dem Mittelpositionsort des Bewegungszyklus.

4. Verfahren (400, 500, 600) nach Anspruch 1 oder 2, wobei das Verfahren zum Bestimmen der dominanten Bewegung (403, 503, 603) in Bezug zu dem Referenzort Bestimmen einer Bildregistrierung jedes der ursprünglichen volumetrischen 3D-Bilder zu dem Referenzort umfasst.

5. Verfahren (400, 500, 600) nach Anspruch 4, wobei Bestimmen einer Bildregistrierung eines ursprünglichen volumetrischen 3D-Bildes zu dem Referenzort eines von Folgendem umfasst:
Berechnen einer direkten Bildregistrierung zwischen dem ursprünglichen volumetrischen 3D-Bild und einem volumetrischen 3D-Bild, das den Referenzort repräsentiert;
Zusammensetzen einer Bildregistrierung von dem ursprünglichen volumetrischen 3D-Bild zu einem zweiten volumetrischen 3D-Bild mit einer Registrierung von dem zweiten volumetrischen 3D-Bild zu dem Referenzort.

6. Verfahren (400, 500, 600) nach Anspruch 3, wobei der Mittelpositionsort des Bewegungszyklus bestimmt wird, umfassend die folgenden Schritte:
Auswählen eines volumetrischen 3D-Bildes, das an einem Ort innerhalb des Bewegungszyklus erfasst wird;
Registrieren mindestens eines der ursprünglichen volumetrischen 3D-Bilder zu dem ausgewählten volumetrischen 3D-Bild unter Verwendung von Bildregistrierung;
Bestimmen einer Registrierung durch Berechnen des Mittelwerts aller Bildregistrierungen zwischen dem ausgewählten volumetrischen 3D-Bild und den ursprünglichen volumetrischen 3D-Bildern.

7. Verfahren (400, 500, 600) nach einem der vorhergehenden Ansprüche, wobei die Schätzung der Sekundärbewegung (404, 504, 604) innerhalb der volumetrischen medizinischen 3D-Bilder räumlich variiert.

8. Verfahren (400, 500, 600) nach einem der vorhergehenden Ansprüche, wobei die volumetrischen medizinischen 3D-Bilder Bilder des Abdomen- oder Thoraxraums sind, und die dominante Bewegung mindestens eine von Atembewegung und Herzbewegung ist, und die Sekundärbewegung mindestens eine von Atembewegung und Herzbewegung ist.

9. Verfahren (400, 500, 600) nach einem der vorhergehenden Ansprüche, wobei Schätzen der Sekundärbewegung (404, 504, 604) gemäß mindestens einem von Folgendem durchgeführt wird:
einer Schätzung von Unschärfe oder Schärfe in dem volumetrischen medizinischen 3D-Bild;
regionaler Bildintensität im volumetrischen medizinischen 3D-Bild;
einer Messung der Differenz zwischen dem volumetrischen medizinischen 3D-Bild und einer geschärften Version des volumetrischen medizinischen 3D-Bildes;
einer Schätzung des Orts einer 2D-Schicht des volumetrischen medizinischen 3D-Bildes innerhalb eines sekundären Bewegungszyklus.

10. Verfahren (400, 500, 600) nach Anspruch 9, wobei die Schätzung des Orts innerhalb des sekundären Bewegungszyklus der 2D-Schicht der volumetrischen medizinischen 3D-Bilder eines oder mehrere der Folgenden umfasst:
Verwenden einer Messvorrichtung zum Messen eines physiologischen Signals, das mit der Sekundärbewegung in Beziehung steht;
Anpassen eines periodischen Signals an zeitlich geordnete Daten;
Verwenden von automatischer Bildsegmentierung, um eine Änderung der Anatomie zu bestimmen;
unter Verwendung von Bildverarbeitungstechniken
Verwenden des Zeitstempels der Erfassung der 2D-Schicht;
Verwenden der Größe einer automatisch konturierten anatomischen Region innerhalb der 2D-Schicht.

11. Verfahren (400, 500, 600) nach Anspruch 9, wobei Schätzen der Bildschärfe gemäß mindestens einem der Folgenden erfolgt:
Bildgradienteninformationen, die unter Verwendung von Differentialtechniken berechnet werden;
Bildgradienteninformationen, die unter Verwendung von Frequenztechniken berechnet werden;
Bildgradienteninformationen, die unter Verwendung mathematischer Morphologietechniken berechnet werden;
Bewerten der Differenz zu einer geschärften Version desselben Bildes;
Bewerten des Einflusses inkrementeller Unschärfen auf das ursprüngliche Bild;
Verwenden eines Maschinenlernmodells, um den Grad der Unschärfe/Schärfe zu schätzen.

12. Verfahren (400, 500, 600) nach einem der vorhergehenden Ansprüche, wobei Konstruieren des volumetrischen abgeleiteten 3D-Bildes basierend auf der geschätzten Sekundärbewegung die Schritte umfasst:
Auswählen von Daten aus mindestens einem der bewegungskompensierten volumetrischen medizinischen 3D-Bilder basierend auf der sekundären Bewegungsschätzung;
Kombinieren der Intensitätswerte der ausgewählten Daten bei jedem Voxel;
Einstellen der Intensitätswerte des abgeleiteten volumetrischen 3D-Bildes gemäß den kombinierten Intensitätswerten.

13. Verfahren (400, 500, 600) nach Anspruch 12, wobei das Verfahren zum Auswählen von Daten aus den bewegungskompensierten volumetrischen medizinischen 3D-Bildern mindestens eines der Folgenden umfasst:
Auswählen von Daten aus dem Bild, das mit der niedrigsten sekundären Bewegungsschätzung assoziiert ist;
Auswählen von Daten aus den N Bildern, die mit der niedrigsten sekundären Bewegungsschätzung assoziiert sind, wobei die Anzahl N vordefiniert ist;
Auswählen von Daten aus den Bildern, die mit einer sekundären Bewegungsschätzung assoziiert sind, die niedriger als eine vordefinierte Schwelle ist;
Auswählen von Daten aus den Bildern, die mit einer sekundären Bewegungsschätzung assoziiert sind, die niedriger als eine automatisch bestimmte Schwelle ist; und wobei das Auswahlverfahren gemäß dem räumlichen Ort innerhalb des abgeleiteten volumetrischen medizinischen 3D-Bildes variiert.

14. Verfahren (400, 500, 600) nach Anspruch 12 oder 13, wobei das Verfahren zum Kombinieren der Intensitätswerte für die Auswahldaten mindestens eines von Folgenden umfasst:
den Mittelwert der Intensitäten;
den Medianwert der Intensitäten;
eine zentrale Tendenzschätzung der Intensitäten;
den Mittelwert der Intensitäten, gewichtet nach ihrer sekundären Bewegungsschätzung;
den Medianwert der Intensitäten, gewichtet nach ihrer sekundären Bewegungsschätzung;
eine zentrale Tendenzschätzung der nach ihrer sekundären Bewegungsschätzung gewichteten Intensitäten.

15. Bildgebungssystem zum Erzeugen eines volumetrischen 3D-Bildes aus volumetrischen 4D-Zeitserien-Bilddaten, umfassend Mittel zum Durchführen des Verfahrens nach den Ansprüchen 1-14.

## Revendications

1. Procédé mis en œuvre par ordinateur (400, 500, 600) permettant de créer une image dérivée volumétrique 3D à compensation de mouvement d'une image médicale volumétrique 4D comprenant les étapes suivantes :
acquérir une image médicale volumétrique 4D (401, 501, 601) comprenant une pluralité d'images médicales volumétriques 3D formant une série chronologique d'images médicales volumétriques 3D représentant des phases d'un cycle de mouvement physiologique ;
déterminer un mouvement dominant (403, 503, 603) de chaque image médicale volumique 3D par rapport à un emplacement de référence qui représente une position par rapport au cycle de mouvement ;
rééchantillonner chacune des images médicales volumétriques 3D par rapport à l'emplacement de référence pour compenser le mouvement dominant déterminé ;
estimer un mouvement secondaire (404, 504, 604) dans au moins une de chaque image médicale volumétrique 3D à compensation de mouvement ou de chacune des images médicales volumétriques 3D originales ;
et
construire une image dérivée volumétrique 3D (405, 505, 605) par combinaison de données provenant des images volumétriques 3D à compensation de mouvement sur la base du mouvement secondaire estimé.

2. Procédé (400, 500, 600) selon la revendication 1 dans lequel l'estimation du mouvement secondaire (404, 504, 604) comprend la détermination du mouvement secondaire dans chacune des images médicales volumétriques 3D à compensation de mouvement et chacune des images médicales volumétriques 3D originales.

3. Procédé (400, 500, 600) selon la revendication 1 ou 2 dans lequel l'emplacement de référence est l'un parmi :
l'emplacement dans le cycle de mouvement de l'une des images médicales 3D originales ;
un extremum du cycle de mouvement ;
une combinaison géométrique des emplacements dans le cycle de mouvement des images médicales 3D originales ;
l'emplacement de la position médiane du cycle de mouvement.

4. Procédé (400, 500, 600) selon la revendication 1 ou 2 dans lequel le procédé de détermination du mouvement dominant (403, 503, 603) par rapport à l'emplacement de référence comprend la détermination d'un recalage d'image de chacune des images volumétriques 3D originales par rapport à l'emplacement de référence.

5. Procédé (400, 500, 600) selon la revendication 4 dans lequel la détermination d'un recalage d'image d'une image volumétrique 3D originale par rapport à l'emplacement de référence comprend un élément parmi :
le calcul d'un recalage direct d'image entre l'image volumétrique 3D originale et une image volumétrique 3D représentant l'emplacement de référence ;
la composition d'un recalage d'image de l'image volumétrique 3D originale par rapport à une seconde image volumétrique 3D avec un recalage de la seconde image volumétrique 3D par rapport à l'emplacement de référence.

6. Procédé (400, 500, 600) selon la revendication 3 dans lequel l'emplacement de position médiane du cycle de mouvement est déterminé comprenant les étapes suivantes ; sélectionner une image volumétrique 3D acquise à un emplacement dans le cycle de mouvement ;
recaler, à l'aide du recalage d'image, au moins une des images volumétriques 3D originales par rapport à l'image volumétrique 3D sélectionnée ;
déterminer un recalage en calculant la moyenne de tous les recalages d'images entre l'image volumétrique 3D sélectionnée et les images volumétriques 3D originales.

7. Procédé (400, 500, 600) selon une quelconque revendication précédente dans lequel l'estimation du mouvement secondaire (404, 504, 604) varie spatialement à l'intérieur des images médicales volumétriques 3D.

8. Procédé (400, 500, 600) selon une quelconque revendication précédente dans lequel les images médicales volumétriques 3D sont des images de la cavité abdominale ou thoracique et le mouvement dominant est au moins un parmi un mouvement respiratoire et un mouvement cardiaque, et le mouvement secondaire est au moins un parmi un mouvement respiratoire et un mouvement cardiaque.

9. Procédé (400, 500, 600) selon une quelconque revendication précédente dans lequel l'estimation du mouvement secondaire (404, 504, 604) est effectuée selon au moins l'une parmi :
une estimation du flou ou de la netteté dans l'image médicale volumétrique 3D ;
l'intensité régionale de l'image dans l'image médicale volumétrique 3D ;
une mesure de la différence entre l'image médicale volumétrique 3D et une version nette de l'image médicale volumétrique 3D ;
une estimation de l'emplacement d'une coupe 2D de l'image médicale volumétrique 3D dans un cycle de mouvement secondaire.

10. Procédé (400, 500, 600) selon la revendication 9 dans lequel l'estimation de l'emplacement dans le cycle de mouvement secondaire de la coupe 2D des images médicales volumétriques 3D comprend un ou plusieurs parmi :
l'utilisation d'un dispositif de mesure pour mesurer un signal physiologique lié au mouvement secondaire ;
l'ajustement d'un signal périodique à des données temporelles ordonnées ;
l'utilisation d'une segmentation automatique d'images pour déterminer un changement d'anatomie ;
l'utilisation de techniques de traitement d'images
l'utilisation de l'estampille temporelle de l'acquisition de la coupe 2D ;
l'utilisation de la taille d'une région anatomique profilée automatiquement dans la coupe 2D.

11. Procédé (400, 500, 600) selon la revendication 9 dans lequel l'estimation de la netteté de l'image est effectuée selon au moins l'une parmi :
des informations de gradient d'image calculées à l'aide de techniques différentielles ;
des informations de gradient d'image calculées à l'aide de techniques fréquentielles ;
des informations de gradient d'image calculées à l'aide de techniques mathématiques de morphologie ;
l'évaluation de la différence par rapport à une version nette de la même image ;
l'évaluation de l'impact d'un flou incrémentiel sur l'image originale ;
l'utilisation d'un modèle d'apprentissage automatique pour estimer le degré de flou/netteté.

12. Procédé (400, 500, 600) selon une quelconque revendication précédente dans lequel la construction de l'image dérivée volumétrique 3D sur la base du mouvement secondaire estimé comprend les étapes suivantes ;
la sélection de données à partir d'au moins l'une des images médicales volumétriques 3D à compensation de mouvement sur la base de l'estimation de mouvement secondaire ;
la combinaison des valeurs d'intensité des données sélectionnées au niveau de chaque voxel ;
le réglage des valeurs d'intensité de l'image volumétrique 3D dérivée selon les valeurs d'intensité combinées.

13. Procédé (400, 500, 600) selon la revendication 12 dans lequel le procédé de sélection de données à partir des images médicales volumétriques 3D à compensation de mouvement comprend au moins l'une parmi :
la sélection de données à partir de l'image associée à l'estimation de mouvement secondaire la plus basse,
la sélection de données à partir des N images associées à l'estimation de mouvement secondaire la plus basse, le nombre N étant prédéfini ;
la sélection de données à partir des images associées à une estimation de mouvement secondaire inférieure à un seuil prédéfini ;
la sélection de données à partir des images associées à une estimation de mouvement secondaire inférieure à un seuil déterminé automatiquement ; et dans lequel le procédé de sélection varie selon l'emplacement spatial à l'intérieur de l'image médicale volumétrique 3D dérivée.

14. Procédé (400, 500, 600) selon la revendication 12 ou 13 dans lequel le procédé de combinaison des valeurs d'intensité pour les données de sélection comprend au moins l'une parmi :
la moyenne des intensités ;
la médiane des intensités ;
une estimation de tendance centrale des intensités ;
la moyenne des intensités pondérées selon leur estimation de mouvement secondaire ;
la médiane des intensités pondérées selon leur estimation de mouvement secondaire ;
une estimation de tendance centrale des intensités pondérées selon leur estimation de mouvement secondaire.

15. Système d'imagerie permettant de créer une image volumétrique 3D à partir de données d'images volumétriques 4D de série chronologique comprenant des moyens pour réaliser le procédé selon les revendications 1 à 14.
